# EUROPÄISCHE PATENTANMELDUNG

(11) **EP 1 197 242 A1**
(43) Veröffentlichungstag der Anmeldung: **17.04.2002**
(21) Anmeldenummer: 00122467.4
(22) Anmeldetag: 13.10.2000
(51) Int. Cl.: A61M 39/24

(54) **Venenverweilkanüle**

(71) Anmelder: Arrabona Medical KFT., 1173 Budapest (HU)
(72) Erfinder: Wechler, Ingolf Max, 1173 Budapest (HU)
(74) Vertreter: Klunker . Schmitt-Nilson . Hirsch

(57) **Zusammenfassung**

Infusionsvorrichtung, bestehend aus einer Venenverweilkanüle mit einem Katheterhalter, einem Kunststoffkatheter und einem Stahlkanülenhalter mit einer den Kunststoffkatheter und den Katheterhalter durchsetzenden Stahlkanüle, wobei in einem zentral das Gehäuse des Katheterhalters durchsetzenden Kanal ein schlauchförmiger Ventilkörper aus einem elastomeren Kunststoff mit einer bestimmten Länge L angeordnet ist und die Wände des Ventilkörpers über die bestimmte Länge L aufeinanderliegen und sich direkt berühren, wobei die bestimmte Länge L, die Wandstärke und die Shore-Härte des Ventilkörpers so aufeinander abgestimmt sind, daß eine Infusionslösung unter Wirkung der Schwerkraft das Ventil passieren kann, daß aber ein Rückfluß von Patientenblut zuverlässig verhindert wird. Vorzugsweise ist der Ventilkörper ein Folienschlauch.

## Beschreibung

Die vorliegende Erfindung betrifft eine Infusionsvorrichtung, wie sie üblicherweise in der Medizintechnik verwendet wird, um einem Patienten Lösungen oder Blutersatzmittel per Infusion zuzuführen. Infusionsvorrichtungen der hier in Betracht kommenden Art bestehen aus einer Venenverweilkanüle, d. h. einem Kathetenhalter mit einem Katheter aus flexiblem Kunststoff wie PTFE und einem an dem Katheterhalter angeordneten Stahlkanülenhalter mit Stahlkanüle, wobei die Stahlkanüle axial durch die Venenverweilkanüle bzw. durch den Katheterhalter und die Kunststoffkanüle hindurchführt, so daß ihre Spitze an dem entfernten Ende der Kunststoffkanüle hervorsteht. Der Stahlkanülenhalter ist mit einer Luer-Lock-Verbindung, einer Art Schraubdeckel, an dem der Stahlkanüle gegenüberliegenden axialen Ende abgeschlossen. Die Luer-Lock-Verbindung kann entfernt werden, um eine Infusionsleitung an dem Stahlkanülenhalter anzuschließen. Eine derart vorbereitete Infusionsvorrichtung wird in eine Vene eingestochen und möglichst weit in die so punktierte Vene vorgeschoben. Der Katheterhalter wird sodann mittels flexibler Laschen auf der Haut des Patentienten befestigt und der Stahlkanülenhalter kann anschließend zusammen mit der Stahlkanüle vollständig aus der Venenverweilkanüle zurückgezogen werden. In der Vene verbleibt lediglich die mit dem außerhalb des Körpers fixierten Katheterhalter verbundenen Kunststoffkanüle. Statt an dem Stahlkanülenhalter kann nun die Infusionsleitung direkt an den Katheterhalter angeschlossen werden.

Wenn die Stahlkanüle abgezogen wird, besteht eine freie Verbindung zwischen der Körpervene und dem Inneren der Verweilkanüle bzw. dem Inneren des Kanals, der das Gehäuse des Katheterhalters durchsetzt. Bis zum Anschluß einer Infusionsleitung an den Katheterhalter kann daher Blut austreten. Das gleiche gilt natürlich beim Wechseln der Infusionsleitung.

Dieser Blutaustritt muß aus Gründen des Patientenschutzes sowie auch aus Gründen des Schutzes des Betreuungspersonals, beispielsweise der Krankenpfleger, der Krankenschwestern und Ärzte verhindert werden. Direkte Blutkontakte sind im Hinblick auf eine mögliche Infektion (HIV, Hepatitis) unbedingt zu vermeiden.

Es ist bereits bekannt, zur Vermeidung dieser Gefahren in dem Kanal, der das Gehäuse des Kathetenhalters durchsetzt, ein Rückschlagventil vorzusehen. Die bekannten Rückschlagventile sind jedoch aus vielen Einzelteilen aufgebaut, entsprechend schwierig zu montieren und auch entsprechend störungsanfällig (DE 38 09 127 und DE 41 37 019).

Die der Erfindung zugrundeliegende Aufgabe besteht darin, eine Infusionsvorrichtung und insbesondere eine Venenverweilkanüle zu schaffen, die einen einfachen Aufbau hat und damit kostengünstig hergestellt werden kann. Trotz der Einfachheit des Aufbaus soll die Venenverweilkanüle die Gefahr eines unbeabsichtigten Austritts von Patientenblut zuverlässig verhindern.

Erfindungsgemäß wird die Aufgabe durch die Merkmale der unabhängigen Ansprüche 1 und 8 gelöst. Besonders bevorzugte Ausbildungen und Ausgestaltungen der erfindungsgemäßen Venenverweilkanüle sind Gegenstand der Unteransprüche.

Nachfolgend werden Ausführungsformen der Erfindung anhand der beigefügten Zeichnung beispielsweise beschrieben. Darin zeigen:
- Fig. 1: die Schnittansicht einer Infusionsvorrichtung nach dem Stand der Technik;
- Fig. 2: die Schnittansicht durch das Gehäuse eines Katheterhalters gemäß einer ersten Ausführungsform der Erfindung;
- Fig. 3A: die perspektivische Ansicht eines Ventilkörpers gemäß einer zweiten Ausführungsform der Erfindung;
- Fig. 3B: die Schnittansicht des Ventilkörpers des in der Fig. 3A gezeigten Ventilkörpers;
- Fig. 4A: die Schnittansicht durch das Gehäuse eines Katheterhalters gemäß einer dritten Ausführungsform der Erfindung;
- Fig. 4B und 4C: Modifikationen des Ventilkörpers gemäß der Fig. 4A;
- Fig. 5A: die Schnittansicht durch eine weitere Modifikation des Ventilkörpers gemäß einer vierten Ausführungsform der Erfindung;
- Fig. 5B: den Ventilkörper gemäß der Fig. 5A mit einer zum Austritt der Infusionslösung geöffneten Klappe;

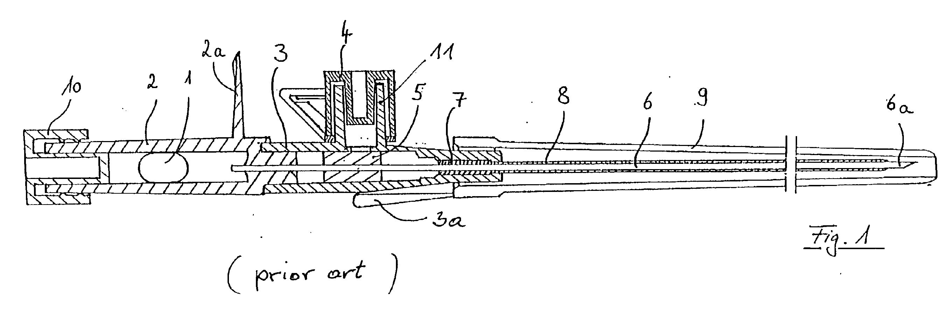

In Fig. 1 ist eine Infusionsvorrichtung gemäß dem Stand der Technik schematisch dargestellt. Die Infusionsvorrichtung besteht aus einem Katheterhalter 3, an dessen rechtem Ende ein Kunststoffkatheter 8, vorzugsweise aus flexiblem Kunststoff wie PTFE, mittels einer in dem Katheterhalter angeordneten Fixierhülse 7 befestigt ist. An dem anderen axialen Ende des Katheterhalters 3 ist ein Stahlkanülenhalter 2 mit einer Stahlkanüle 6 angeordnet, die den Katheterhalter 3 und den Kunststoffkatheter 8 axial vollständig durchdringt und aus der axial gegenüberliegenden Seite des Kunststoffkatheters 8 hervorsteht. Der Stahlkanülenhalter 2 ist an der der Stahlkanüle 6 axial gegenüberliegenden Seite durch eine Luer-Lock-Verbindung 10 abgeschlossen. In dem Stahlkanülenhalter 2 ist ein Porex-Filter 1 vorgesehen. Außerdem besitzt der Stahlkanülenhalter 2 eine nach oben ragende Angriffsplatte 2a, an der der Verwender der Infusionsvorrichtung z. B. mit dem Daumen angreifen kann, um eine Blutbahn mit der Infusionsvorrichtung zu punktieren und dieselbe in die punktierte Blutbahn vorzuschieben. Der Katheterhalter ist mit einem seitlich angeordneten Zuspritzstutzen 11 ausgestattet, der im nichtbenutzten Zustand durch eine Kappe 4 verschlossen ist.

Außerdem sind als weitere Bestandteile des Stahlkanülenhalters 3 in dessen Unterseite seitlich hervorstehende Klappen 3a vorgesehen, die nach der Punktion flach auf der Haut des Patienten aufliegen und z. B. mit Pflaster an der Haut befestigt werden können. Die Stahlkanüle mit dem sie umgebenden Kunststoffkatheter ist im unbenutzten Zustand der Infusionsvorrichtung durch eine Kappe 9 geschützt.

Mit 5 ist ein Ventilkörper bezeichnet, der eine Ventilfunktion für den radial angeordneten Zuspritzstutzen 11 übernimmt.

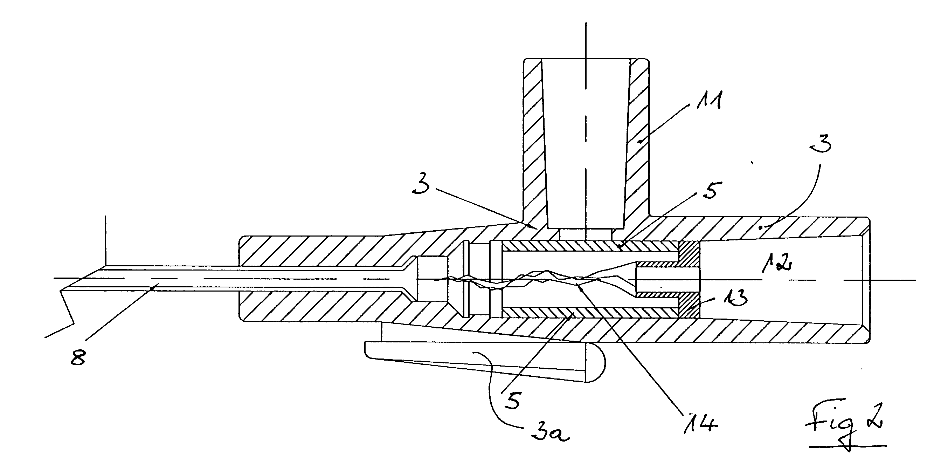

In Fig. 2 ist eine erste Ausführungsform der Erfindung dargestellt. Im Kanal 12 des Katheterhalters 3 ist ein Stutzen 13 dichtend eingesetzt, auf dessen patientenseitiges Ende ein Ventilkörper in Form eines Folienschlauchs 14 aufgezogen ist. Der Folienschlauch besteht vorzugsweise aus einem dünnwandigen elastischen Material, z. B. HDPE (high density polyethylene). Die Länge des Folienschlauchs kann beispielsweise 10 mm betragen, die Breite des flach aufliegenden Folienschlauchs etwa 2,5 mm.

Der Folienschlauch ist in Fig. 2 in dem Zustand gezeigt, den er einnimmt, wenn die Stahlkanüle mit dem Stahlkanülenhalter aus dem Katheterhalter 3 abgezogen ist. Das in Fig. 2 rechts liegende freie Ende des Kanals 12 wird mit einer Infusionseinrichtung verbunden, der Katheter 8 ist im betriebsbereiten Zustand der Vorrichtung in eine Vene des Patienten eingeführt. Im Lagerzustand (nicht gezeigt) durchsetzt die Stahlkanüle den Folienschlauch.

Die den Katheterhalter von rechts nach links durchlaufende Infusionsflüssigkeit durchdringt den Folienschlauch 14 ohne Schwierigkeiten. Das treibende Druckgefälle ist der Differenzdruck zwischen dem hydrostatischen Druck der Infusionsflüssigkeit und dem Blutdruck des Patienten.

Es ist ohne weiteres ersichtlich, daß der Folienschlauch zwar von rechts nach links, nicht aber von links nach rechts durchflossen werden kann. Er wird vielmehr bei abgetrennter Infusionseinrichtung den Folienschlauch zusammengedrückt und verhindert so jeglichen Rückfluß.

Selbstverständlich muß die Wandstärke des Folienschlauchs, seine Länge und seine Elastizität bzw. Shore-Härte so aufeinander abgestimmt sein, daß der gewünschte Effekt auch eintritt, d. h. daß einerseits Infusionsflüssigkeit unter Wirkung der Schwerkraft den Folienschlauch durchsetzen kann, daß aber andererseits ein Rückfluß von Patientenblut zuverlässig verhindert wird. Eine Abstimmung dieser Parameter ist aber ohne größere Schwierigkeiten mit der Durchführung einiger weniger Versuche möglich.

Der Ventilkörper 5 besteht aus einer zylindrischen Hülse aus einem elastomeren Material. Die Hülse verschließt die Zuspritzöffnung des seitlichen Zuspritzstutzens 11. Wenn durch diesen Stutzen zusätzliche Medikamente eingespritzt werden sollen, weicht der Ventilkörper 5 im Bereich der Durchtrittsöffnung des Stutzens 11 in das Gehäuse 3 in Richtung der Mittelachse des Kanals 12 aus und gibt den Weg für die durch diesen Stutzen eingespritzte Flüssigkeit frei. Im Ruhezustand nimmt der Ventilkörper die in der Fig. 2 gezeigte Position ein und verschließt die Auslaßöffnung zum Zuspritzstutzen 11 zuverlässig, so daß auch durch diesen Stutzen kein Patientenblut austreten kann.

Der Folienschlauch 14 kann auf den Stutzen 11 einfach aufgeschoben werden. Möglich ist aber auch ein Verkleben oder Verschweißen mittels Ultraschall.

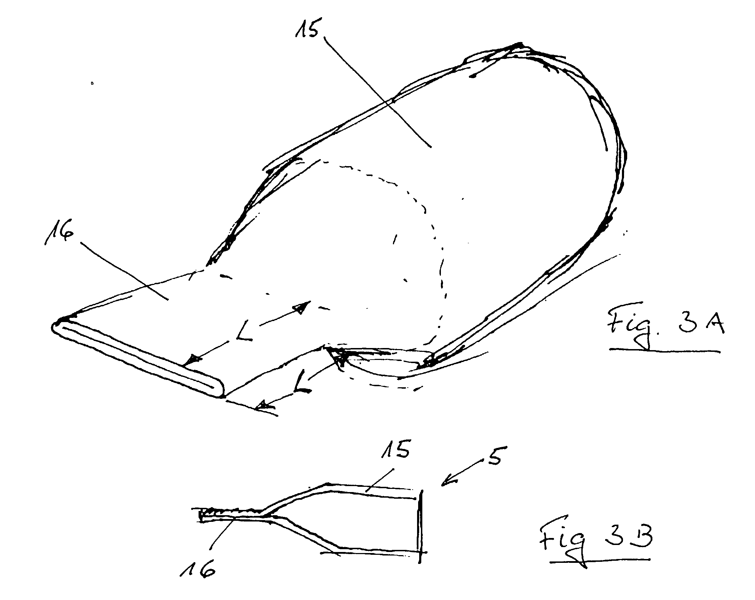

Die Fig. 3A zeigt nun in perspektivischer Ansicht eine Abwandlung des Ventilkörpers, wie er in Fig. 2 dargestellt ist. Statt des Folienschlauches 14 wird der Ventilkörper 5 derart modifiziert, daß er einerseits einen zylindrischen Bereich 15 aufweist, der patientenseitig zu einem flachen Bereich 16 zusammengedrückt ist. Dieser flache Bereich hat eine bestimmte Länge L. Im Bereich dieser Länge liegen die Wände des Ventilkörpers aufeinander, sie bilden eine Art "Entenschnabel".

Es ist ohne weiteres ersichtlich, daß durch diesen flachen Bereich bestimmter Länge zwar Infusionsflüssigkeit von rechts nach links hindurchtreten kann, daß aber ein Rückfluß von Patientenblut nicht möglich ist, da das unter Druck stehende Blut selbst den Bereich 16 hermetisch schließt, wenn der Gegendruck der Infusionsflüssigkeit gegen Null geht.

Abweichend von der Ausführungsform der Erfindung wie sie in Fig. 2 gezeigt ist, benötigt man hier also keinen separaten Folienschlauch, sondern es wird der Ventilkörper 5, der ohnehin erforderlich ist, um den seitlichen Zuspritzstutzen abzudichten, so weitergebildet, wie es in der Fig. 3 gezeigt ist. Damit übernimmt der Zylinderbereich 15 des Ventilkörpers 5 die bereits bekannte Ventilfunktion für den zeitlichen Zuspritzstutzen, wogegen der flache Bereich 16 mit der Länge L, der "Entenschnabel", die Ventilfunktion für die eintretende Infusionsflüssigkeit und die Absperrung des rückfließenden Patientenbluts übernimmt.

Vorzugsweise besteht der Ventilkörper aus Silikon, das ohne weiteres mittels Spritzguß in eine Form gebracht werden kann, wie sie in der Fig. 3 gezeigt ist. Natürlich muß die Wandstärke des flachen Bereichs 16, sowie die Länge des flachen Bereichs 16 und die Shore-Härte des verwendeten Materials so aufeinander abgestimmt werden, daß die beschriebene Ventilfunktion zuverlässig erfüllt wird.

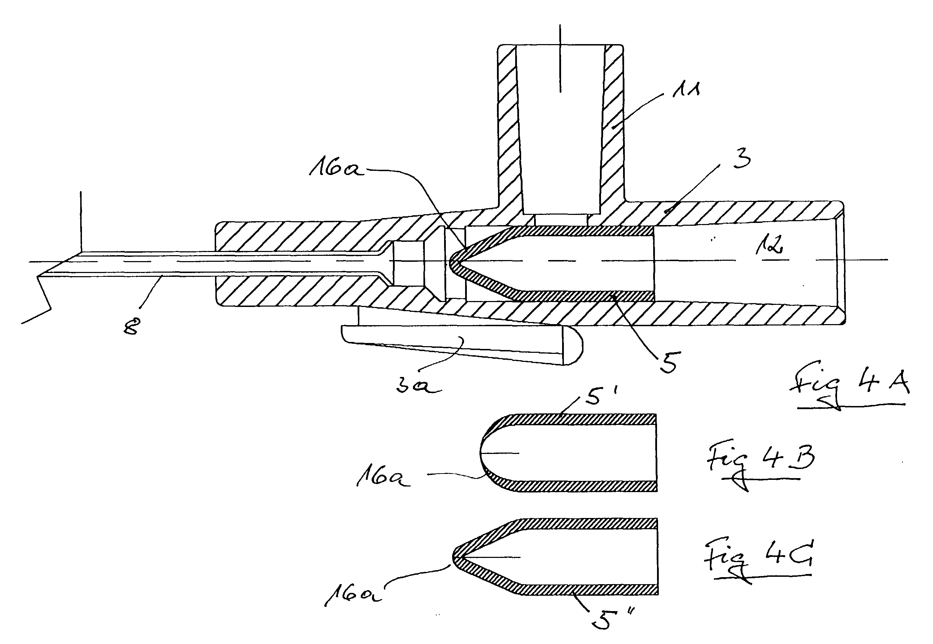

Fig. 4 zeigt nun eine dritte Ausführungsform der Erfindung, bei welcher der Ventilkörper 5 patientenseitig mit einer Kappe verschlossen ist, die kegel- oder kugelförmig ausgebildet sein kann. Die Kappe ist, ausgehend von ihrem Scheitelpunkt, seitlich geschlitzt, wobei die Schlitze etwa radial verlaufen. Die derart geschlitzten Kappen ermöglichen den Durchtritt von Infusionsflüssigkeit unter einem relativ geringen Differenzdruck, wogegen ein umgekehrter Durchfluß der Kappen, d. h. von Patientenblut, in der Figur von rechts nach links, nicht möglich ist, bzw. einen wesentlich höheren Differenzdruck erfordert, der in der Praxis nicht vorliegt. Selbstverständlich müssen die Wandstärken des Materials, seine Shore-Härte und die Anordnung der Schlitze so gewählt werden, daß der gewünschte Effekt auch eintritt. Wie der in Fig. 4B gezeigte Ventilkörper zeigt, können die Wandstärken im Bereich der Kappe auch reduziert sein. Die Fig. 4C zeigt nochmals den in der Fig. 4A im eingebauten Zustand dargestellten Ventilkörper. Die Kappe dieses Ventilkörpers ist kegelig oder aber nach Art eines Dachkantprismas keilförmig zulaufend. Wie schon bei der Ausführungsform nach Fig. 3 wird auch der in der Fig. 4 gezeigte Ventilkörper vorzugsweise im Spritzgußverfahren aus Silikon hergestellt.

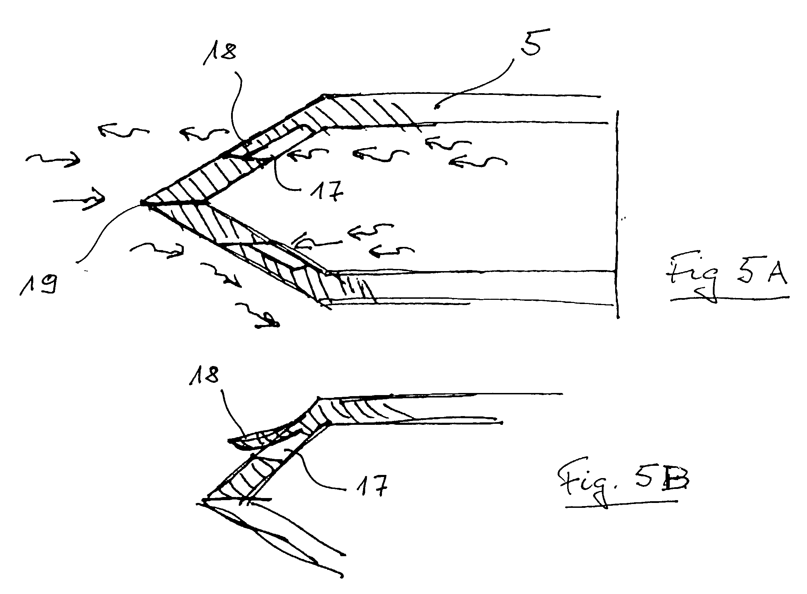

In der Fig. 5 ist eine Modifikation des im Zusammenhang mit der Fig. 4 erläuterten Ventilkörpers 5 gezeigt. Der in Fig. 5 dargestellte Ventilkörper hat eine relativ hohe Wandstärke und eine Endkappe, die nach Art eines Dachkantprismas in einem Scheitel zusammenläuft, die für den Durchtritt der Stahlkanüle geschlitzt ist. Dieser Schlitz stellt kein Ventil im Sinne der Erfindung dar, d. h. er dient nach entfernter Stahlkanüle nicht mehr dem Durchtritt von Infusionsflüssigkeit, sondern schließt unter relativ hohem Schließdruck hermetisch ab. Der Durchtritt der Infusionsflüssigkeit erfolgt durch in die Endkappe eingestanzte Fensteröffnungen 17, die durch Klappen 18 verschlossen sind. Vorzugsweise sind die Klappen 18 mit verringerter Wandstärke ausgeführt, so daß sie sich leicht öffnen können, wie dies schematisch in Fig. 5B dargestellt ist.

Nach dem Entfernen der Stahlkanüle wird sich also der zentrale Schlitz 19 der Kappe 16a hermetisch schließen. Die hohe Schließkraft dieses Durchlasses ist insoweit vorteilhaft, als die Infusionsvorrichtung oftmals über längere Zeiträume hinweg mit eingeführter Stahlkanüle gelagert werden muß. Die lange Lagerzeit führt leicht zu Materialermüdungen, d. h. Verringerung der Elastizität und damit der Rückstellkraft des Ventils. Bei dieser Ausführungsform kann jedoch mit so hohen Rückstellkräften gearbeitet werden, daß in jedem Fall ein zuverlässiges Verschließen des Schlitzes 19 erfolgt.

Der Durchtritt der Infusionsflüssigkeit von rechts nach links in der Fig. 5 ist durch Pfeile angedeutet und erfolgt durch die sich leicht patientenseitig öffnenden Klappen 18. Umgekehrt preßt zurückströmendes Patientenblut die Klappen 18 fest in die Fensteröffnungen 17 und verschließen diese, so daß ein Durchtritt von Blut durch das Ventil zuverlässig verhindert wird.

Selbstverständlich muß auch bei dieser Ausführungsform durch eine Abstimmung von Wandstärke, Ausbildung und Shore-Härte des Materials die gewünschte Ventilfunktion optimiert werden.

Es ist offensichtlich, daß statt des in Fig. 5 dargestellten Dachkantprismas die Kappe 16a des Ventilkörpers 5 auch in Form einer Halbkugel oder kegelförmig ausgebildet sein kann. Wie bei den anderen Ausführungsformen kann auch der Ventilkörper dieser Ausführungsform im Spritzgußverfahren aus Silikon hergestellt werden. Die Fensteröffnungen 17 sowie die verringerte Wandstärke der Klappen 18 wird vorzugsweise anschließend durch Stanzen und Schneiden bzw. Fräsen erzeugt.

Zusammengefaßt bietet die Erfindung eine verblüffend einfache Lösung für ein Rückschlagventil, das trotz langer Lagerzeit mit eingeführter Stahlkanüle, d. h. im geöffneten Zustand, einwandfrei funktioniert.

## Patentansprüche

1. Infusionsvorrichtung, bestehend aus einer Venenverweilkanüle mit einem Katheterhalter (3), einem Kunststoffkatheter (8) und einem Stahlkanülenhalter (2) mit einer den Kunststoffkatheter (8) und den Katheterhalter (3) durchsetzenden Stahlkanüle (6), wobei in einem zentral das Gehäuse des Katheterhalters (3) durchsetzenden Kanal (12) ein schlauchförmiger Ventilkörper (14, 15) aus einem elastomeren Kunststoff mit einer bestimmten Länge L angeordnet ist und die Wände des Ventilkörpers (14, 15) über die bestimmte Länge L aufeinanderliegen und sich direkt berühren, wobei die bestimmte Länge L, die Wandstärke und die Shore-Härte des Ventilkörpers (5) so aufeinander abgestimmt sind, daß eine Infusionslösung unter Wirkung der Schwerkraft das Ventil passieren kann, daß aber ein Rückfluß von Patientenblut zuverlässig verhindert wird.

2. Infusionsvorrichtung nach Anspruch 1, dadurch gekennzeichnet, daß der Ventilkörper (5) auf das patientenseitige Ende eines rohrförmigen Stutzens (13) aufgezogen ist, der in den Kanal (12) des Katheterhalters (3) eingesetzt ist.

3. Infusionsvorrichtung nach Anspruch 2, **dadurch gekennzeichnet, daß** der Ventilkörper (5) ein Folienschlauch (14) ist.

4. Infusionsvorrichtung nach Anspruch 3, **dadurch gekennzeichnet, daß** der Folienschlauch aus HDPE ist.

5. Infusionsvorrichtung nach Anspruch 3 oder 4, **dadurch gekennzeichnet, daß** der Folienschlauch (14) im kollabierten Zustand eine Breite von 2,5 mm und eine Länge von etwa 10 mm hat.

6. Infusionsvorrichtung nach Anspruch 1, **dadurch gekennzeichnet, daß** der Ventilkörper (5) einen selbsttragenden zylindrischen Bereich (15) aufweist, der dichtend in den den Katheterhalter (3) durchsetzenden Kanal (12) eingesetzt ist und der zylindrische Bereich patientenseitig in einen flachen Bereich (16) bestimmter Länge L übergeht, in welchem die Wände aufeinanderliegen und dieser Bereich die Ventilfunktion liefert.

7. Infusionsvorrichtung nach Anspruch 6, **dadurch gekennzeichnet, daß** die Wandstärke im flachen Bereich (16) geringer ist, als im zylindrischen Bereich (15).

8. Infusionsvorrichtung bestehend aus einer Venenverweilkanüle mit einem Katheterhalter (3), einem Kunststoffkatheter (8) und einem Stahlkanülenhalter (2), wobei in einem zentral in das Gehäuse des Katheterhalters (3) durchsetzenden Kanal (12) ein selbsttragender hülsenförmiger Ventilkörper (5) aus einem elastomeren Kunststoff dichtend eingesetzt ist, der an seinem patientenseitigen Ende eine Endkappe (16a) aufweist, die wenigstens einen Schlitz aufweist, wobei Wandstärke, Shore-Härte und axiale Länge der Schlitze so aufeinander abgestimmt sind, daß eine Infusionslösung unter Wirkung der Schwerkraft das Ventil passieren kann, daß aber ein Rückfluß von Patientenblut zuverlässig verhindert wird.

9. Infusionsvorrichtung nach Anspruch 1, **dadurch gekennzeichnet, daß** der Ventilkörper (5) in seinem zylindrischen Bereich (15) als Ventil für einen radial angeordneten Zuspritzstutzen (11) dient.

10. Infusionsvorrichtung nach Anspruch 8 oder 9, **dadurch gekennzeichnet, daß** die Kappe (16a) kugel- oder kegelförmig ist und die Schlitze, ausgehend von ihrem Scheitelpunkt, im wesentlichen radial angeordnet sind.

11. Infusionsvorrichtung nach einem der Ansprüche 8 bis 10, **dadurch gekennzeichnet, daß** in der Endkappe (16a) Fensteröffnungen (17) teilweise freigestanzt sind, die durch sich in Richtung des Patienten öffnende Klappen (18) verschlossen sind, wobei die Fensterform, die Wandstärke der Klappen (18) und die Shore-Härte des Kappenmaterials so aufeinander abgestimmt sind, daß eine Infusionslösung unter Wirkung der Schwerkraft die Fensteröffnungen passieren kann, daß aber ein Rückfluß von Patientenblut zuverlässig verhindert wird.

12. Infusionsvorrichtung nach Anspruch 11, **dadurch gekennzeichnet, daß** die Endkappe (16a) neben den Fenstern (17) nur einen zentralen Schlitz (19) aufweist, der dem Durchtritt der Stahlkanüle (6) dient und der nach dem Entfernen der Stahlkanüle (6) wieder schließt und keine weitere Ventilfunktion übernimmt.

13. Infusionsvorrichtung nach einem oder mehreren der Ansprüche 6 bis 12, **dadurch gekennzeichnet, daß** der Ventilkörper (5) aus Silikon besteht.

14. Infusionsvorrichtung nach Anspruch 3, **dadurch gekennzeichnet, daß** der Ventilkörper ein Spritzgußteil ist.
